(19) Europäisches Patentamt
European Patent Office
Office européen des brevets

(11) EP 2 483 276 B1

(12) **FASCICULE DE BREVET EUROPEEN**

(45) Date de publication et mention de la délivrance du brevet:
**02.11.2016 Bulletin 2016/44**

(21) Numéro de dépôt: **10776772.5**

(22) Date de dépôt: **30.09.2010**

(51) Int Cl.:
***C07D 493/04*** (2006.01)

(86) Numéro de dépôt international:
**PCT/FR2010/052066**

(87) Numéro de publication internationale:
**WO 2011/039483 (07.04.2011 Gazette 2011/14)**

(54) **PROCÉDÉ DE PRÉPARATION DE DI (ALKYLCARBONATE) DE DIANHYDROHEXITOL**

VERFAHREN ZUR HERSTELLUNG EINES DIALKYLCARBONATS AUS DIANHYDROHEXITOL

METHOD FOR PREPARING A DIALKYL CARBONATE OF DIANHYDROHEXITOL

(84) Etats contractants désignés:
**AL AT BE BG CH CY CZ DE DK EE ES FI FR GB GR HR HU IE IS IT LI LT LU LV MC MK MT NL NO PL PT RO SE SI SK SM TR**

(30) Priorité: **01.10.2009 FR 0956835**

(43) Date de publication de la demande:
**08.08.2012 Bulletin 2012/32**

(73) Titulaire: **Roquette Frères**
**62136 Lestrem (FR)**

(72) Inventeurs:
- **FUERTES, Patrick**
**F-59160 Lomme (FR)**
- **IBERT, Mathias**
**F-59930 La Chapelle D'armentieres (FR)**
- **JOSIEN, Emilie**
**F-62350 St Venant (FR)**
- **TUNDO, Pietro**
**I-30174 Mestre-venezia (IT)**
- **ARICÒ, Fabio**
**I-35141 Padova (IT)**

(74) Mandataire: **Cabinet Plasseraud**
**66, rue de la Chaussée d'Antin**
**75440 Paris Cedex 09 (FR)**

(56) Documents cités:
**US-A1- 2004 241 553**

- **OKADA, MASAHIKO ET AL: "Biodegradable polymers based on renewable resources. VI. Synthesis and biodegradability of poly(ester carbonate)s containing 1,4:3,6-dianhydro-D-glucitol and sebacic acid units", 2002, JOURNAL OF APPLIED POLYMER SCIENCE , 86(4), 872-880 CODEN: JAPNAB; ISSN: 0021-8995, XP002582390, page 878**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 483 276 B1

## Description

La présente invention concerne un nouveau procédé de préparation de dialkylcarbonates de 1,4 : 3,6-dianhydrohexitols, certains nouveaux dialkylcarbonates obtenus par ce procédé et l'utilisation des dialkylcarbonates de dianhydrohexitols pour la préparation de polymères de synthèse, en particulier en tant que (co)monomères pour la synthèse de polycarbonates et de polycarbamates, ou en tant qu'agent d'allongement de chaîne pour l'augmentation de la masse moléculaire de polymères contenant des groupes terminaux réactifs susceptibles de réagir avec des fonctions carbonate.

Les polycarbonates sont des matériaux thermoplastiques amorphes, classiquement obtenus par polycondensation de diols et de carbonate de diphényle, de phosgène ou de diphosgène.

La toxicité du phosgène, du diphosgène ou du phénol, formé inévitablement en cas d'utilisation de carbonate de diphényle, constitue un inconvénient important de la synthèse des polycarbonates.

Le développement de matériaux polymères issus de ressources biologiques renouvelables à court terme est devenu un impératif écologique et économique, face à l'épuisement et à la montée des prix des ressources fossiles telles que le pétrole.

Dans ce contexte, l'utilisation de dianhydrohexitols, issus de (poly)saccharides végétaux, en tant que monomères dihydroxylés dans des réactions de polycondensation, semble être une approche prometteuse de remplacement de monomères d'origine pétrochimiques.

La préparation de polycarbonates à base d'isosorbide a été décrite dans la demande de brevet EP 2 033 981. Ce document décrit la polycondensation d'un mélange d'isosorbide, d'au moins un deuxième diol alicyclique et de carbonate de diphényle. Le procédé présente l'inconvénient, déjà mentionné ci-dessus, de générer du phénol en tant que sous-produit de la réaction de polymérisation.

L'article de Saber CHATTI, intitulé « Cyclic and Noncyclic Polycarbonates of Isosorbide (1,4 :3,6-dianhydro-D-glucitol), dans Macromolecules, 2006, 9061-9070, envisage différentes voies de synthèse de polycondensats à base d'isosorbide. Une première voie de synthèse, consistant à chauffer de l'isosorbide en présence de quatre équivalents molaires de carbonate de diméthyle ou diéthyle et en présence d'un catalyseur choisi parmi le tertiobutylate de potassium (KOtBu), le dioctanoate d'étain ($SnOct_2$) et le tétrabutoxyde de titane ($Ti(OBu)_4$), à des températures comprises entre 100 °C et 200 °C, est décrite comme ne permettant pas l'obtention de polycarbonates d'isosorbide. D'après les auteurs de cet article, de l'isosorbide n'ayant pas réagi est récupéré au bout de plus de deux heures de réaction à 200 °C. Cet échec a été confirmé par la Demanderesse qui a observé que le chauffage d'un mélange d'isosorbide et de carbonate de diméthyle en présence de tertiobutylate de potassium, de dioctanoate d'étain ou de tétrabutoxyde de titane aboutissait à des mélanges comprenant une forte proportion d'alkyléthers d'isosorbide et une proportion faible, voire nulle, de méthylcarbonate d'isosorbide. Ces trois catalyseurs s'avèrent ainsi être également ou uniquement des catalyseurs d'éthérification et non pas uniquement des catalyseurs de transestérification comme souhaité dans la présente invention. Ils ne permettent pas de former sélectivement des dialkylcarbonates de dianhydrohexitols et donc, indirectement, ne sont pas utilisables en vue de la préparation de polymères (polycarbonates).

L'article de Saber Chatti décrit deux autres voies de synthèse qui souffrent toutefois toutes les deux de l'inconvénient de nécessiter l'emploi de réactifs ou solvants toxiques voire très toxiques (phosgène, diphosgène, pyridine, bis-chloroformiate d'isosorbide).

La préparation de dialkylcarbonates de dianhydrohexitols a également été décrite dans la demande de brevet US 2004/241553. Ce document décrit un procédé de fabrication de di(alkylcarbonate) de dianhydrohexitol par réaction d'un dianhydrohexitol et d'un ester chloroformique. Ce procédé de fabrication présente l'inconvénient majeur de faire intervenir un composé toxique, i.e. un ester chloroformique.

Le document JP 6-261774 décrit également un procédé de préparation de di(alkylcarbonate) de dianhydrohexitol (exemple 5). Cependant, ce procédé de préparation fait ici encore intervenir des espèces chloroformiques toxiques. La présente invention est basée sur la découverte que certains catalyseurs sélectionnés, utilisés par ailleurs dans des conditions réactionnelles particulières, permettaient d'obtenir avec des rendements presque quantitatifs et en l'absence d'utilisation de réactifs toxiques des di(alkylcarbonate) de dianhydrohexitols dont certains n'ont jusqu'ici jamais été isolés.

La Demanderesse entend ici par « presque quantitatifs » des rendements en dialkylcarbonate de dianhydrohexitol supérieurs à 70%, préférentiellement supérieurs ou égaux à 75%, et plus préférentiellement encore supérieurs ou égaux à 80%, ou des rendements en dialkylcarbonate de dianhydrohexitol et en oligomères supérieurs à 90%. Le fait de disposer, grâce au nouveau procédé de l'invention, de dialkylcarbonates de dianhydrohexitols sous forme pure ou presque pure, permet de préparer des polycarbonates à base de dianhydrohexitols, en particulier à base d'isosorbide, sans utilisation de réactifs toxiques. Les diéthylcarbonates de dianhydrohexitols de la présente invention permettent même la synthèse de polycarbonates ou de polycarbamates sans aucune émission de produits secondaires toxiques tels que le phénol ou, dans une moindre mesure, le méthanol.

La présente invention a par conséquent pour objet un procédé de préparation de dialkylcarbonates de dianhydrohexitols de formule (I)

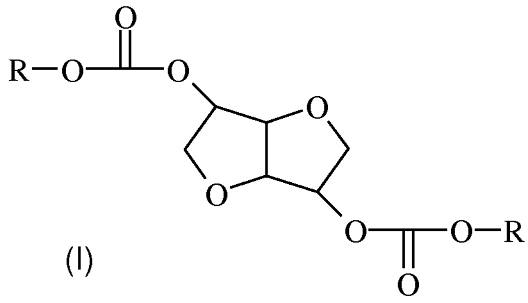

(I)

où chaque R représente indépendamment un groupe alkyle linéaire ou ramifié, de préférence un groupe alkyle en $C_{1-6}$, en particulier un groupe méthyle ou éthyle, ledit procédé comprenant, dans l'ordre, les étapes suivantes :

(a) préparation d'un mélange réactionnel initial contenant

- au moins un dianhydrohexitol,
- au moins 2 équivalents en moles, rapportés à la quantité de dianhydrohexitol présent, d'au moins un carbonate de di(alkyle) de formule R-O-C(=O)-O-R où R a la signification indiquée ci-dessus, et
- un catalyseur de transestérification,

(b) chauffage du mélange réactionnel jusqu'à une température supérieure ou égale à la température d'ébullition de l'alcool R-OH formé par la réaction de transestérification, ou supérieure ou égale à la température d'ébullition du mélange azéotropique que forme l'alcool R-OH obtenu avec un autre des composants présents dans le mélange réactionnel, et au plus égale à la température d'ébullition du mélange réactionnel, dans un réacteur muni de préférence d'une colonne de rectification comportant un nombre de plateaux théoriques de distillation suffisant pour séparer du mélange réactionnel l'alcool obtenu, ou l'azéotrope qu'il forme avec un autre des composants présents dans le mélange réactionnel.

Le terme « 1,4 : 3,6-dianhydrohexitol » ou « dianhydrohexitol », utilisé dans la présente invention, englobe l'isosorbide (obtenu par déshydratation du D-glucitol), l'isomannide (obtenu par déshydratation du D-mannitol) et l'isoidide (obtenu par déshydratation du D-iditol).

Le mélange réactionnel préparé à l'étape (a) peut contenir un ou plusieurs dianhydrohexitols mais contient de préférence un seul dianhydrohexitol, en particulier de l'isosorbide, disponible en plus grande quantité et à plus faible coût que les deux autres stéréoisomères.

La réaction au coeur de la présente invention est une réaction de transestérification. On comprendra aisément que, pour aboutir à une fraction satisfaisante de dérivés difonctionnels, c'est-à-dire de molécules de dianhydrohexitols dont les deux fonctions hydroxyle ont été converties en fonctions alkylcarbonate R-O-(C=O)-O-, il est nécessaire d'introduire dans le milieu réactionnel initial au moins autant de moles de carbonate de dialkyle que de fonctions hydroxyle portées par le dianhydrohexitol, autrement dit deux fois plus de moles de carbonate de dialkyle que de moles de dianhydrohexitol.

L'utilisation d'un rapport molaire 2:1 (carbonate de dialkyle:dianhydrohexitol) n'est toutefois généralement pas optimale. En effet, le monoalkylcarbonate de dianhydrohexitol et le di(alkylcarbonate) de dianhydrohexitol formés au cours de la réaction peuvent réagir avec du dianhydrohexitol n'ayant pas encore réagi ce qui aboutit à la formation de dimères ou d'oligomères. Cette formation de dimères ou d'oligomères peut être inhibée efficacement par l'utilisation d'un excès important de carbonate de dialkyle. Les exemples d'application ci-après montrent en effet que plus l'excès de carbonate de diéthyle ou de carbonate de diméthyle est important, plus la fraction d'oligomères est faible. Un excès molaire supérieur à 40 permet de limiter la fraction oligomérique à environ 5%, préférentiellement à environ 1 %.

L'utilisation d'un très grand excès de carbonate de dialkyle n'est toutefois pas dépourvue d'inconvénients. En effet, elle implique toujours une augmentation indésirable de l'installation de production. Par ailleurs, plus l'excès de carbonate de diéthyle utilisé au départ est important, plus il faudra mettre en oeuvre d'énergie pour éliminer, à la fin de la réaction de transestérification, ce réactif du produit obtenu par évaporation. Il convient par conséquent de trouver, pour chaque cas, un compromis entre une teneur optimale en dialkylcarbonate de dianhydrohexitol qui nécessite un grand excès de carbonate de dialkyle et une réduction toujours souhaitable des coûts de production.

Le mélange réactionnel initial préparé à l'étape (a) contient par conséquent avantageusement de 2,1 à 100 équivalents molaires, de préférence de 5 à 60 équivalents molaires, et en particulier de 10 à 40 équivalents molaires de carbonate de dialkyle, rapportés à la quantité de dianhydrohexitol.

Lorsque, comme c'est le cas du méthanol, l'alcool généré par la réaction de transestérification forme avec le

carbonate de diméthyle de départ un mélange azéotropique, l'évaporation de ce mélange « consomme » du réactif. Il va de soit que dans un tel cas, le nombre minimal d'équivalents de carbonate de dialkyle est supérieur à la valeur théorique de 2. Bien entendu, ce nombre minimal dépendra des proportions du mélange azéotropique et est d'autant plus important que la fraction de carbonate de dialkyle dans le mélange azéotropique est élevée. Ainsi, pour le mélange azéotropique MeOH/DMC (70/30) le nombre minimal d'équivalents de DMC qu'il est nécessaire d'introduire dans le mélange réactionnel initial est égal à 2,9.

Le catalyseur de transestérification utilisé dans le procédé de la présente invention est de préférence choisi parmi un ou plusieurs des suivants :

- les catalyseurs acides,
- les carbonates et hydrogénocarbonates de métaux alcalins et alcalino-terreux,
- les hydroxydes de métaux alcalins et alcalino-terreux,
- les phosphates, hydrogénophosphates et dihydrogénophosphates de métaux alcalins et alcalino-terreux,
- les sels d'ammonium choisis parmi les carbonates, hydrogénocarbonates, hydroxydes, phosphates, hydrogénophosphates et dihydrogénophosphates d'ammonium, et
- les amines, en particulier les amines aromatiques et les alkylamines.

Les sels d'ammonium en question peuvent être des sels d'ammonium primaire, secondaire, tertiaire ou quaternaire.

L'amine peut être primaire, secondaire ou tertiaire, en particulier secondaire ou tertiaire.

Le mélange réactionnel initial préparé à l'étape (a) contient de préférence de 0,1 à 10 équivalents molaires, en particulier de 1 à 5 équivalents molaires, et tout particulièrement de 1 à 3 équivalents molaires de catalyseur de transestérification, rapportés à la quantité de dianhydrohexitol.

On peut citer en particulier, à titre de catalyseurs de transestérification, l'acide sulfurique, l'acide para-toluènesulfonique, l'acide phosphorique, le carbonate de potassium, le carbonate de sodium, le carbonate de baryum, le carbonate de césium, l'hydrogénocarbonate de potassium, l'hydrogénocarbonate de sodium, l'hydroxyde de lithium, l'hydroxyde de potassium, l'hydroxyde de sodium, l'hydroxyde de calcium, les hydrotalcites, le phosphate de sodium, le phosphate de potassium, la pyridine, la triéthylamine et la diisopropylamine.

De manière encore plus préférentielle, le catalyseur de transestérification utilisé dans le procédé de la présente invention est choisi parmi un ou plusieurs des catalyseurs suivants, exclusivement spécifiques de la réaction de transestérification selon l'invention: le carbonate de potassium, l'hydroxyde de potassium et l'hydroxyde de lithium.

Ces catalyseurs présentent également l'avantage d'être insolubles dans le milieu réactionnel et de réaliser une catalyse en phase hétérogène. Ils sont donc récupérables par un simple procédé de filtration et sont par conséquent facile à recycler.

Le mélange réactionnel initial peut éventuellement contenir un solvant organique, par exemple un solvant organique formant un mélange azéotropique avec l'alcool R-OH, par exemple le méthanol ou l'éthanol, formé au cours de la réaction. Dans un mode de réalisation préféré du procédé de l'invention, le mélange réactionnel initial ne contient toutefois pas d'autres solvants ou composants que ceux mentionnés ci-dessus et est de préférence constitué d'au moins un 1,4 : 3,6-dianhydrohexitol, d'un carbonate de dialkyle, et du catalyseur de transestérification.

Le mélange réactionnel est ensuite introduit dans un réacteur muni d'un système d'agitation, éventuellement d'une colonne de rectification et d'un système de chauffage et est chauffé à une température qui est

- soit supérieure ou égale à la température d'ébullition de l'alcool R-OH formé par la réaction de transestérification,
- soit supérieure ou égale à la température d'ébullition de l'azéotrope que forme l'alcool R-OH avec un composant du milieu réactionnel, tel que le carbonate de dialkyle ou un solvant ajouté.

La « température d'ébullition » est bien entendu celle du solvant ou azéotrope à la pression de l'installation.

On entend ici par « température de chauffage » la température mesurée par un capteur thermique dans le mélange réactionnel. La réaction de transestérification est d'autant plus rapide que la température de chauffage est élevée. Il est toutefois bien entendu impossible de chauffer le mélange réactionnel au-delà de la température d'ébullition du mélange réactionnel. Il est particulièrement avantageux de mettre en oeuvre la réaction de transestérification à une température égale à la température d'ébullition du milieu réactionnel ou inférieure tout au plus de quelques degrés à cette dernière, par exemple inférieure d'au plus 2 °C à cette dernière.

Il peut être intéressant d'augmenter la pression de fonctionnement du système à une valeur supérieure à la pression atmosphérique. Ceci pourrait en effet améliorer la vitesse de réaction (grâce à l'augmentation des températures d'ébullition) ou augmenter la différence de température entre l'alcool ou l'azéotrope formé et le carbonate de dialkyle de départ ce qui permettrait de simplifier la séparation par rectification et de réduire le nombre de plateaux théoriques de la colonne de rectification.

On peut envisager par exemple de travailler à une pression absolue comprise entre 0 et 20 bars.

Lorsqu'on fait réagir un dianhydrohexitol avec du carbonate de diméthyle (DMC), le méthanol obtenu forme avec le DMC un mélange azéotropique dont la température d'ébullition est égale à 62,7 °C. Le point d'ébullition du DMC est égal à 90 °C. Ainsi, lorsqu'on met en oeuvre le procédé selon l'invention avec du DMC, la température de chauffage est comprise entre environ 68 °C et 95 °C, de préférence entre 85 et 90 °C.

L'éthanol (point d'ébullition 78,3 °C), contrairement au méthanol, ne forme pas de mélange azéotropique avec le carbonate de diéthyle (DEC). Par conséquent, lorsqu'on met en oeuvre le procédé selon l'invention en partant du DEC, la température de chauffage est comprise entre environ 83 °C et 130 °C, de préférence entre 121 et 126 °C.

Afin d'optimiser la conduite du procédé de l'invention, il est utile de suivre le degré d'avancement de la réaction et d'arrêter le chauffage dès que le degré d'avancement est atteint. Le procédé selon l'invention comprend par conséquent en outre le suivi de l'avancement de la réaction. On peut suivre par exemple la quantité d'alcool formé ou bien surveiller la température des vapeurs au niveau de la tête de colonne de rectification. Lorsque celle-ci augmente rapidement de la température d'ébullition de l'alcool, ou du mélange azéotropique contenant l'alcool, à la température d'ébullition du carbonate de dialkyle, c'est le signe que la réaction est terminée, généralement suite à la consommation de l'ensemble des groupes hydroxyle du dianhydrohexitol.

Le degré d'avancement de la réaction d'estérification mise en oeuvre dans la présente invention est défini par la formule suivante :

$$\text{Degré d'avancement (en \%)} = \frac{(Ni - Nt)}{Ni} x100$$

où

*Nt* = nombre de fonctions hydroxyle de dianhydrohexitol restant dans le milieu réactionnel au temps t, et
*Ni* = nombre de fonctions hydroxyle initial de dianhydrohexitol.

Le chauffage du mélange réactionnel (à l'étape (b)) est avantageusement poursuivi jusqu'à ce qu'un degré d'avancement de la réaction d'au moins 95 %, de préférence d'au moins 98 %, et idéalement égal à 100 % soit atteint.

Il est important de noter qu'un degré d'avancement égal à 100 % ne signifie pas que le mélange réactionnel contienne uniquement du dialkylcarbonate de dianhydrohexitol. En effet, en particulier lorsqu'on utilise un rapport molaire carbonate de dialkyle/dianhydrohexitol relativement faible, par exemple inférieur égal à 20 ou 10, le mélange réactionnel contient une certaine fraction d'oligomères (oligocarbonates). Ces oligomères n'ont pas forcément besoin d'être séparés du produit réactionnel principal visé qu'est le dialkylcarbonate de dianhydrohexitol. En effet pour certaines applications, par exemple pour la synthèse de polycarbonates ou d'autres polymères de synthèse, les oligomères peuvent remplir une fonction équivalente à celle du dialkylcarbonate de dianhydrohexitol.

Le procédé selon l'invention comprend de préférence en outre au moins une étape de séparation du dialkylcarbonate de dianhydrohexitol obtenu, d'avec une partie ou la totalité des autres composants du mélange réactionnel.

Dans le cas de l'utilisation d'un catalyseur solide non soluble dans le milieu réactionnel, une première étape de séparation est généralement la filtration du milieu réactionnel, destinée à éliminer et recycler le catalyseur solide en suspension dans ledit milieu.

Le mélange réactionnel débarrassé des particules de catalyseur est ensuite soumis à une évaporation, de préférence sous vide, du carbonate de dialkyle n'ayant pas réagi. Après évaporation du carbonate de dialkyle on obtient soit un produit solide que l'on pourra recristalliser une ou plusieurs fois dans un solvant approprié. C'est le cas du diméthylcarbonate d'isosorbide, du diméthylcarbonate d'isomannide et du diméthylcarbonate d'isoidide qui peuvent être recristallisés par exemple dans de l'isopropanol.

Lorsque, comme c'est le cas des dérivés diéthylcarbonate, le produit réactionnel est liquide après évaporation du carbonate de dialkyle, il peut être séparé des oligomères ou des dérivés monoéthylcarbonate, par distillation.

Selon un mode de réalisation préféré de la présente invention, la séparation du dialkylcarbonate d'anhydrohexitol d'avec les oligomères peut être réalisée sur un évaporateur à surface raclée en configuration « short path ». Le dialkylcarbonate d'anhydrohexitol est évaporé tandis que les oligomères sont récupérés dans le résidu non évaporé.

Ainsi, la présente invention a avantageusement pour objet un procédé de préparation de dialkylcarbonates de dianhydrohexitol caractérisé en ce qu'il comprend en outre une étape (c) de concentration du ou des dialkylcarbonates de dianhydrohexitol obtenus à l'étape (b) par évaporation du dialkylcarbonate n'ayant pas réagi au cours de ladite étape (b).

La présente invention concerne également un procédé de préparation de dialkylcarbonates de dianhydrohexitol caractérisé en ce qu'il comprend en outre une étape (d) de purification du ou des dialkylcarbonates de dianhydrohexitol concentrés à l'étape (c).

Selon un mode de réalisation particulier de la présente invention, le résidu riche en oligomères peut avantageusement être recyclé vers le mélange réactionnel. Ainsi, par exemple, ledit résidu est réintroduit dans le réacteur en présence d'une quantité minimale d'alcanol. La Demanderesse a mis en évidence, dans ces conditions particulières, la réversibilité de la réaction de transestérification selon l'invention. Un tel ajout du résidu riche en oligomères en tête du réacteur induit la production de dianhydrohexitol et de carbonate de di(alkyle) de telle sorte que les conditions adéquates pour la réaction de préparation de di(alkylcarbonate) de dianhydrohexitol selon l'invention soient à nouveau réunies.

Ainsi, la présente invention concerne avantageusement un procédé de préparation de dialkylcarbonates de dianhydrohexitol caractérisé en ce que le résidu issu de l'étape (d) de purification est recyclé vers le mélange réactionnel de l'étape (a).

Bien que le procédé selon l'invention puisse être mis en oeuvre en principe indifféremment et avec une égale chance de réussite avec les trois dianhydrohexitols mentionnés en introduction, on préfère parmi ceux-ci, pour des raisons de disponibilité et de coût, l'isosorbide.

En choisira de préférence en tant que carbonate de dialkyle, le carbonate de diéthyle.

A la connaissance de la Demanderesse seul le diméthylcarbonate d'isosorbide a jusqu'ici été produit et isolé (JP 6-261774, exemple 5).

Au contraire, les diéthylcarbonates de dianhydrohexitol, à savoir le diéthylcarbonate d'isosorbide, le diéthylcarbonate d'isomannide et le diéthylcarbonate d'isoidide, ainsi que le di(méthylcarbonate) d'isoidide et le di(méthylcarbonate) d'isomannide n'ont jusqu'ici pas été isolés et constituent par conséquent un autre objet de l'invention. Parmi ces nouveaux composés, les diéthylcarbonates de dianhydrohexitols et en particulier le di(éthylcarbonate d'isosorbide) sont considérés comme particulièrement intéressants, car non seulement leur préparation selon le procédé de l'invention peut être mise en oeuvre en l'absence d'un quelconque composé organique volatil toxique, mais l'utilisation de ces dérivés de dianhydrohexitols pour la synthèse de polymères présente l'avantage de ne libérer en principe que de l'éthanol, c'est-à-dire un composé organique considéré comme non toxique.

La variante du procédé selon l'invention aboutissant à la synthèse des diéthylcarbonates de dianhydrohexitols permet ainsi, pour la première fois, d'envisager la synthèse de polycarbonates à base d'isosorbide, ou plus généralement à base de dianhydrohexitols, sans utilisation ni formation d'aucun composé toxique, ce qui est un progrès considérable par rapport aux procédés proposés jusqu'ici.

Les dialkylcarbonate de dianhydrohexitols préparés selon la présente invention permettent en outre de synthétiser des copolymères alternés à base de dianhydrohexitol. Une telle synthèse n'a jusqu'ici pas été possible. En effet, la synthèse de copolymères à base de dianhydrohexitols décrite dans EP 2 033 981 comprend la réaction d'un mélange de dianhydrohexitol, d'un deuxième composé dihydroxylé, alicyclique, et d'un dicarbonate en présence d'un catalyseur de transestérification. Cette synthèse aboutit toutefois à l'obtention de copolymères statistiques et non pas de copolymères alternés ayant une répartition homogène des motifs co-monomères tout au long de la chaîne.

Par conséquent, la présente invention a également pour objet l'utilisation d'un ou plusieurs des dialkylcarbonates de dianhydrohexitols préparés selon le procédé de l'invention en tant que monomères ou comonomères pour la préparation de polycarbonates, de polycarbamates ou de polythiocarbonates, de préférence pour la préparation de polycarbonates ou polycarbamates alternés.

La présente invention a en outre pour objet l'utilisation d'un des composés selon l'invention ou d'un composé susceptible d'être obtenu par le procédé selon l'invention, en tant qu'agent d'allongement de chaîne, agent de pontage, agent de greffage et agent de réticulation de polymères portant des fonctions latérales ou terminales à hydrogène labile, susceptible de réagir avec une fonction carbonate, c'est-à-dire des fonctions hydroxyle, amine, thiol, ester ou oxazoline.

**Exemple 1 : Préparation de di(méthylcarbonate) d'isosorbide selon l'invention**

On introduit dans un réacteur d'une capacité de 1,5 litres, chauffé par un bain thermostaté à fluide caloporteur, muni d'un système d'agitation mécanique à pale, d'un système de contrôle de la température du milieu réactionnel et d'une colonne de rectification surmontée d'une tête de reflux, 43 g d'isosorbide (0,29 mol) puis 1069 g de carbonate de diméthyle (= 40 équivalents molaires par rapport à l'isosorbide) et 123 g de carbonate de potassium. On chauffe le mélange réactionnel pendant une heure à reflux total, temps au bout duquel la température des vapeurs en tête de colonne atteint 64°C, avant de débuter l'élimination du méthanol formé. On maintient ensuite le chauffage du milieu réactionnel à une température comprise entre 68 °C et 95 °C pendant 13 heures, temps au bout duquel la température des vapeurs en tête de colonne atteint 90 °C et se stabilise à cette température (point d'ébullition du carbonate de diméthyle). C'est le signe que la réaction de transestérification est terminée et qu'il ne se forme plus de méthanol. On filtre le milieu réactionnel afin d'en éliminer le carbonate de potassium en suspension. La solution limpide et incolore contient 95 % de di(méthylcarbonate) d'isosorbide, 1 % de monométhylcarbonate d'isosorbide et 4 % d'oligomères. Elle est exempte d'isosorbide n'ayant pas réagi. Les pourcentages correspondent à des répartitions de surfaces en analyse chromatographique en phase gazeuse (exclusion faite du solvant, ici le dialkylcarbonate).

**Exemple 2 : Préparation de di(méthylcarbonate) d'isosorbide selon l'invention**

On répète l'Exemple 1 avec la seule différence que l'on utilise non pas 40 équivalents mais seulement 20 équivalents de carbonate de diméthyle. La composition du milieu réactionnel obtenu après élimination des particules de catalyseur est présentée au tableau 1 ci-après.

**Exemple 3 : Préparation de di(méthylcarbonate) d'isosorbide selon l'invention**

On répète l'Exemple 1 avec la seule différence que l'on utilise non pas 40 équivalents mais seulement 10 équivalents de carbonate de diméthyle. La composition du milieu réactionnel obtenu après élimination des particules de catalyseur est présentée au tableau 1 ci-après.

**Exemple 4 : Préparation de di(éthylcarbonate) d'isosorbide selon l'invention**

On introduit dans un réacteur identique à celui utilisé dans l'Exemple 1, 25 g d'isosorbide (0,17 mol) puis 826 g de carbonate de diéthyle (= 40 équivalents molaires par rapport à l'isosorbide) et 70 g de carbonate de potassium. On chauffe le mélange réactionnel pendant une heure à reflux total, avant de débuter l'élimination de l'éthanol formé. On maintient ensuite le chauffage du milieu réactionnel à une température de 126°C pendant 3 heures, temps au bout duquel la température des vapeurs en tête de colonne atteint 126 °C (point d'ébullition du carbonate de diéthyle). C'est le signe que la réaction de transestérification est terminée et qu'il ne se forme plus d'éthanol. On filtre le milieu réactionnel afin d'en éliminer le carbonate de potassium en suspension. La solution obtenue, limpide et incolore, contient 96,6 % de di(éthylcarbonate) d'isosorbide, 0,3 % de monoéthylcarbonate d'isosorbide et 3,1 % d'oligomères. Elle est exempte d'isosorbide n'ayant pas réagi. Les pourcentages correspondent à des répartitions de surfaces en analyse chromatographique en phase gazeuse (exclusion faite du solvant, ici le diéthylcarbonate).

**Exemple 5 : Préparation de di(éthylcarbonate) d'isosorbide selon l'invention**

On répète l'Exemple 4 avec la seule différence que l'on utilise non pas 40 équivalents mais seulement 10 équivalents de carbonate de diéthyle. La composition du milieu réactionnel obtenu après élimination des particules de catalyseur est présentée au tableau 1 ci-après.

Tableau 1

| Ex. | Carbonate de dialkyle | Carbonate de dialkyle /isosorbide | Isosorbide | Monoalkylcarbonate d'isosorbide | Dialkylcarbonate d'isosorbide | oligomères |
|---|---|---|---|---|---|---|
| 1 | DMC | 40 | 0 % | 1 % | 95 % | 4 % |
| 2 | DMC | 20 | 0 % | 3 % | 85 % | 11 % |
| 3 | DMC | 10 | 0 % | 2 % | 80 % | 18 % |
| 4 | DEC | 40 | 0 % | 1 % | 95 % | 4 % |
| 5 | DEC | 10 | 0 % | 8 % | 75 % | 17 % |

Ces résultats montrent que le procédé selon l'invention permet d'obtenir, avec des rendements presque quantitatif, du di(méthylcarbonate) d'isosorbide et du di(éthylcarbonate) d'isosorbide. L'utilisation d'un grand excès stoechiométrique de carbonate de dialkyle permet d'obtenir des produits contenant environ 95 % en poids du dérivé difonctionnel.

Un excès stoechiométrique moins important (10 équivalents) conduit certes à des fractions non négligeable d'oligocarbonates, mais ces derniers peuvent être facilement séparés des dialkylcarbonates d'isosorbide par cristallisation ou distillation.

**Exemple 6 : Préparation de di(méthylcarbonate) d'isosorbide selon l'invention**

On introduit dans un réacteur d'une capacité de 20 litres, chauffé par un bain thermostaté à fluide caloporteur, muni d'un système d'agitation mécanique à pale, d'un système de contrôle de la température du milieu réactionnel et d'une colonne de rectification surmontée d'une tête de reflux, 430 g d'isosorbide (0,29 mol) puis 10,602 kg de carbonate de diméthyle (= 40 équivalents molaires par rapport à l'isosorbide) et 1220 g de carbonate de potassium.

On chauffe le mélange réactionnel à une température d'environ 90°C en éliminant le méthanol formé en continu. Au bout de 5 heures la température des vapeurs en tête de colonne atteint 90 °C et se stabilise à cette température (point d'ébullition du carbonate de diméthyle). C'est le signe que la réaction de transestérification est terminée et qu'il ne se forme plus de méthanol. On filtre le milieu réactionnel afin d'en éliminer le carbonate de potassium en suspension. La solution limpide et incolore contient 95,9 % de di(méthylcarbonate) d'isosorbide, 0,1 % de monométhylcarbonate d'isosorbide et 4 % d'oligomères. Elle est exempte d'isosorbide n'ayant pas réagi. Les pourcentages correspondent à des répartitions de surfaces en analyse chromatographique en phase gazeuse (exclusion faite du solvant).

**Exemple 7 : Purification du di(méthylcarbonate) d'isosorbide obtenu selon l'invention**

Après évaporation du carbonate de diméthyle n'ayant pas réagi, on distille sous vide poussé (<1mBar) 645g du produit obtenu selon l'exemple 6 sur un évaporateur à surface raclée en configuration « short path ». La double enveloppe de l'évaporateur est chauffée à 160°C et le condenseur interne est maintenu à une température d'environ 80°C. Le produit est introduit à 70°C avec un débit de 180 g/h.

On obtient :

- un distillat sous forme d'un solide blanc contenant plus de 99% en poids de di(méthylcarbonate) d'isosorbide et ne contenant aucune trace d'oligomères ; et
- un résidu de distillation contenant 95% en poids d'oligomères.

**Exemple 8 : Réversibilité de la réaction de transestérification selon l'invention**

On introduit 6,8 g des oligomères, correspondant au résidu obtenu selon l'exemple 7, dans un ballon tricol de 250 ml, muni d'un agitateur magnétique, chauffé par un chauffe ballon électrique et surmonté d'un réfrigérant. On introduit ensuite dans ledit ballon tricol 173 g de carbonate de diméthyle, 19,6 g de carbonate de potassium et 3,2 g de méthanol. On maintient le milieu réactionnel 3h au reflux à la température de 90°C. Après filtration du carbonate de potassium, on obtient une solution limpide et incolore contenant 14% de diméthylcarbonate d'isosorbide, 51% de monométhylcarbonate d'isosorbide, 34% d'isosorbide et 1% d'oligomères. Ces pourcentages sont issus d'une analyse par chromatographie en phase gazeuse en répartition de surface (exclusion faite du solvant, ici le diéthylcarbonate).

**Exemple 9 : Etude de la spécificité des catalyseurs de transestérification**

Afin d'évaluer la spécificité des catalyseurs à la réaction de transestérification selon l'invention, on répète l'Exemple 6 avec la seule différence que l'on fait varier le catalyseur comme suit : on utilise soit 3 équivalents de carbonate de potassium comme dans l'Exemple 1, soit 3 équivalents d'hydroxyde de potassium ou d'hydroxyde de lithium, soit de l'hydrotalcite en quantité (poids pour poids) équivalente à la quantité d'isosorbide. L'hydrotalcite utilisée est préalablement calcinée une nuit à 500°C. Il s'agit soit de l'hydrotalcite Sorbacid® 911 ou Sorbacid® 944, toutes deux commercialisées par la société SÜD-CHEMIE.

Après évaporation du carbonate de diméthyle, les pourcentages (en répartition de surface) en diméthylcarbonate d'isosorbide (DMC iso), monométhylcarbonate d'isosorbide (MMC iso), isosorbide, oligomères, diméthylisosorbide (DMI), monométhylisosorbide (MMI) et méthylisosorbideméthylcarbonate (MI-MC) sont determinés par chromatographie en phase gazeuse en répartition de surface (exclusion faite du solvant, ici le diéthylcarbonate).

| Catalyseur | DMC iso | MMC iso | Isosorbide | Oligomères | DMI | MMI | MI-MC |
|---|---|---|---|---|---|---|---|
| LiOH | 95,0 | 0,6 | 0,1 | 4,3 | 0,0 | 0,0 | 0,0 |
| KOH | 83,0 | 12,0 | 0,0 | 5,0 | 0,0 | 0,0 | 0,0 |
| $K_2CO_3$ | 96,0 | 0,1 | 0,0 | 3,9 | 0,0 | 0,0 | 0,0 |
| Sorbacid® 911 | 61,4 | 0,7 | 0,0 | 3,0 | 3,3 | 0,0 | 31,6 |
| Sorbacid® 944 | 73,9 | 2,5 | 0,1 | 8,8 | 0,6 | 0,2 | 13,9 |

Contrairement aux hydrotalcites, le carbonate de potassium, l'hydroxyde de potassium et l'hydroxyde de lithium sont des catalyseurs spécifiques de la réaction de transestérification selon l'invention.

**Exemple 10 : Utilisation des composés selon l'invention pour la préparation d'un polymère**

On introduit dans un réacteur d'une capacité de 100 ml, chauffé par un bain thermostaté à fluide caloporteur, muni d'un système d'agitation mécanique à pale, d'un système de contrôle de la température du milieu réactionnel, d'une tubulure d'introduction d'azote, d'une tête de distillation reliée à un condenseur et à un récipient pour recueillir les condensats, et d'un système de mise sous vide avec régulation, une quantité de 50,6 g (0,19 mole) de diméthylcarbonate d'isosorbide obtenu selon l'exemple 6, 12,3 g (0,20 mole) d'éthylène glycol et 0,2773 g ($8{,}5 \times 10^{-4}$ mole) de carbonate de césium. On met l'installation sous un flux d'azote de 25 l/h et on chauffe le milieu réactionnel par le fluide caloporteur. On monte progressivement la température en effectuant des paliers de 30 mn à 115°C, 140°C et 165°C. La montée en température entre chaque palier se fait en 30 minutes. Au cours de la réaction, on observe une distillation du méthanol. A la fin du palier de 165°C, on met l'installation sous vide pendant 1 h 30 min (pression résiduelle de 3 mbar) afin de poursuivre la distillation et en maintenant la température de 165°C. Après refroidissement du milieu réactionnel, on obtient un polymère visqueux.

## Revendications

**1.** Procédé de préparation de di(alkylcarbonate) de dianhydrohexitol de formule (I)

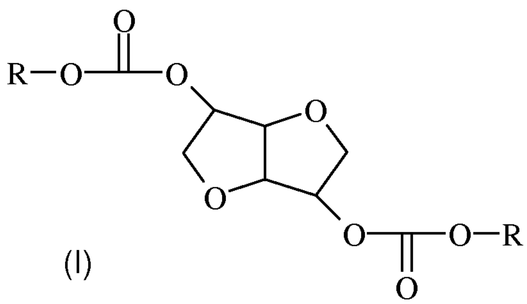

(I)

où chaque R représente indépendamment un groupe alkyle linéaire ou ramifié, de préférence un groupe alkyle en $C_{1-6}$, en particulier un groupe méthyle ou éthyle,
ledit procédé comprenant, dans l'ordre, les étapes suivantes :

(a) préparation d'un mélange réactionnel initial contenant

- au moins un dianhydrohexitol,
- au moins 2 équivalents en moles, rapportés à la quantité de dianhydrohexitol présent, d'au moins un carbonate de di(alkyle) de formule

R-O-C(=O)-O-R

où R a la signification indiquée ci-dessus, et
- un catalyseur de transestérification,

(b) chauffage du mélange réactionnel jusqu'à une température supérieure ou égale à la température d'ébullition de l'alcool R-OH formé par la réaction de transestérification, ou supérieure ou égale à la température d'ébullition du mélange azéotropique que forme l'alcool R-OH obtenu avec un autre des composants présents dans le mélange réactionnel, et au plus égale à la température d'ébullition du mélange réactionnel, dans un réacteur muni d'une colonne de rectification comportant un nombre de plateaux théoriques de distillation suffisant pour séparer du mélange réactionnel l'alcool obtenu, ou l'azéotrope qu'il forme avec un autre des composants présents dans le mélange réactionnel.

**2.** Procédé selon la revendication 1, **caractérisé par le fait que** le mélange réactionnel initial contient de 2,1 à 100 équivalents molaires, de préférence de 5 à 60 équivalents molaires, et en particulier de 10 à 40 équivalents molaires de carbonate de dialkyle, rapportés à la quantité de dianhydrohexitol présent initialement dans le milieu réactionnel.

**3.** Procédé selon la revendication 1 ou 2, **caractérisé par le fait que** le catalyseur de transestérification est choisi parmi :

- les catalyseurs acides,
- les carbonates et hydrogénocarbonates de métaux alcalins et alcalino-terreux,
- les hydroxydes de métaux alcalins et alcalino-terreux,
- les phosphates, hydrogénophosphates et dihydrogénophosphates de métaux alcalins et alcalino-terreux,
- les sels d'ammonium choisis parmi les carbonates, hydrogénocarbonates, hydroxydes, phosphates, hydrogénophosphates et dihydrogénophosphates d'ammonium, et
- les amines.

**4.** Procédé selon l'une quelconque des revendications précédentes, **caractérisé par le fait que** le catalyseur de transestérification est choisi parmi l'acide sulfurique, l'acide para-toluènesulfonique, l'acide phosphorique, le carbonate de potassium, le carbonate de sodium, le carbonate de baryum, le carbonate de césium, l'hydrogénocarbonate de potassium, l'hydrogénocarbonate de sodium, l'hydroxyde de lithium, l'hydroxyde de potassium, l'hydroxyde de sodium, l'hydroxyde de calcium, les hydrotalcites, le phosphate de sodium, le phosphate de potassium, la pyridine, la triéthylamine et la diisopropylamine.

**5.** Procédé selon l'une quelconque des revendications précédentes, **caractérisé par le fait que** le catalyseur de transestérification est choisi parmi le carbonate de potassium, l'hydroxyde de potassium et l'hydroxyde de lithium.

**6.** Procédé selon l'une quelconque des revendications précédentes, **caractérisé par le fait que** le mélange réactionnel initial préparé à l'étape (a) contient de 0,1 à 10 équivalents molaires, de préférence de 1 à 5 équivalents molaires, et en particulier de 1 à 3 équivalents molaires de catalyseur de transestérification, rapportés à la quantité de dianhydrohexitol.

**7.** Procédé selon l'une quelconque des revendications précédentes, **caractérisé par le fait que** le procédé comprend en outre le suivi de l'avancement de la réaction.

**8.** Procédé selon l'une quelconque des revendications précédentes, **caractérisé par le fait que** le chauffage à l'étape (b) est poursuivi jusqu'à un degré d'avancement de la réaction au moins égal à 95 %, de préférence au moins égal à 98 %, et idéalement égal à 100 %, le degré d'avancement de la réaction étant défini par la formule suivante :

$$\text{Degré d'avancement (en \%)} = \frac{(Ni - Nt)}{Ni} x100$$

où

*Nit* = nombre de fonctions hydroxyle de dianhydrohexitol restant dans le milieu réactionnel au temps t, et
*Ni* = nombre de fonctions hydroxyle initial de dianhydrohexitol.

**9.** Procédé selon l'une quelconque des revendications précédentes, **caractérisé par le fait que** le procédé comprend en outre au moins une étape de séparation du dialkylcarbonate de dianhydrohexitol obtenu, d'avec une partie ou la totalité des autres composants du mélange réactionnel.

**10.** Procédé selon l'une quelconque des revendications précédentes, **caractérisé par le fait que** le dianhydrohexitol est l'isosorbide.

**11.** Procédé selon l'une quelconque des revendications précédentes, **caractérisé par le fait que** le carbonate de dialkyle est le carbonate de diéthyle.

**12.** Procédé selon l'une quelconque des revendications précédentes, **caractérisé en ce qu'**il comprend en outre une étape (c) de concentration du ou des dialkylcarbonates de dianhydrohexitol obtenus à l'étape (b) par évaporation du dialkylcarbonate n'ayant pas réagi au cours de ladite étape (b).

**13.** Procédé selon la revendication 12, **caractérisé en ce qu'**il comprend en outre une étape (d) de purification du ou des dialkylcarbonates de dianhydrohexitol concentrés à l'étape (c).

**14.** Procédé selon la revendication 13, **caractérisé en ce que** le résidu issu de l'étape (d) de purification est recyclé vers le mélange réactionnel de l'étape (a).

**15.** Di(éthylcarbonate) de dianhydrohexitol, en particulier le di(éthylcarbonate d'isosorbide).

**16.** Di(méthylcarbonate) d'isoidide.

**17.** Di(méthylcarbonate) d'isomannide.

**18.** Utilisation d'un des composés selon l'une quelconque des revendications 15 à 17 ou d'un composé susceptible d'être obtenu par un procédé selon l'une quelconque des revendications 1 à 14 en tant que monomère ou comonomère pour la préparation de polycarbonates, de polycarbamates ou de polythiocarbonates, de préférence pour la préparation de polycarbonates ou polycarbamates alternés.

**19.** Utilisation d'un des composés selon l'une quelconque des revendications 15 à 17 ou d'un composé susceptible d'être obtenu par un procédé selon l'une quelconque des revendications 1 à 14, en tant qu'agent d'allongement de chaîne, agent de pontage, agent de greffage et agent de réticulation de polymères portant des fonctions hydroxyle, amine, thiol, ester ou oxazoline.

**Patentansprüche**

**1.** Verfahren zur Herstellung von Dianhydrohexitoldi(alkylcarbonat) der Formel (I)

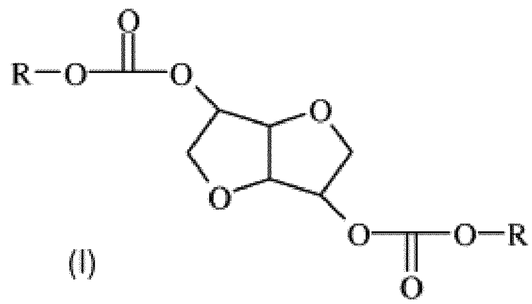

(I) ;

worin jedes R unabhängig eine lineare oder verzweigte Alkylgruppe, vorzugsweise eine $C_{1-6}$-Alkylgruppe, insbesondere eine Methyl- oder Ethylgruppe, darstellt,
wobei das Verfahren die folgenden Schritte in dieser Reihenfolge umfasst:

(a) Herstellung eines Anfangsreaktionsgemisches, enthaltend

- wenigstens ein Dianhydrohexitol,
- wenigstens 2 Moläquivalente, bezogen auf die Menge an vorliegendem Dianhydrohexitol, wenigstens eines Di(alkyl)carbonats der Formel

R-O-C(=O)-O-R,

worin R die oben genannte Bedeutung hat, und
- einen Umesterungskatalysator,

(b) Erhitzen des Reaktionsgemisches bis zu einer Temperatur, die größer oder gleich der Siedetemperatur des durch die Umesterungsreaktion gebildeten Alkohols R-OH ist oder größer oder gleich der Siedetemperatur des azeotropen Gemisches, das der erhaltene Alkohol R-OH mit einer anderen der im Reaktionsgemisch vorliegenden Komponenten bildet, ist und höchstens gleich der Siedetemperatur des Reaktionsgemisches ist, in einem Reaktor mit einer Rektifikationskolonne, welche eine ausreichende Anzahl an theoretischen Destillationsböden aufweist, um den erhaltenen Alkohol oder das Azeotrop, das der Alkohol mit einer anderen der in dem Reaktionsgemisch vorliegenden Komponenten bildet, von dem Reaktionsgemisch abzutrennen.

**2.** Verfahren gemäß Anspruch 1, **dadurch gekennzeichnet, dass** das Anfangsreaktionsgemisch von 2,1 bis 100 molare Äquivalente, vorzugsweise von 5 bis 60 molare Äquivalente, insbesondere von 10 bis 40 molare Äquivalente

an Di(alkyl)carbonat, bezogen auf die Menge des anfangs im Reaktionsmilieu vorliegenden Dianhydrohexitols, enthält.

**3.** Verfahren gemäß Anspruch 1 oder 2, **dadurch gekennzeichnet, dass** der Umesterungskatalysator ausgewählt wird aus:

- sauren Katalysatoren,
- Alkali- und Erdalkalimetallcarbonaten und -hydrogencarbonaten,
- Alkali- und Erdalkalimetallhydroxiden,
- Alkali- und Erdalkalimetallphosphaten, -hydrogenphosphaten und -dihydrogenphosphaten,
- Ammoniumsalzen, ausgewählt aus Ammoniumcarbonaten, -hydrogencarbonaten, -hydroxiden, -phosphaten, -hydrogenphosphaten und -dihydrogenphosphaten, und
- Aminen.

**4.** Verfahren gemäß einem der vorhergehenden Ansprüche, **dadurch gekennzeichnet, dass** der Umesterungskatalysator ausgewählt wird aus Schwefelsäure, para-Toluolsulfonsäure, Phosphorsäure, Kaliumcarbonat, Natriumcarbonat, Bariumcarbonat, Cäsiumcarbonat, Kaliumhydrogencarbonat, Natriumhydrogencarbonat, Lithiumhydroxid, Kaliumhydroxid, Natriumhydroxid, Calciumhydroxid, Hydroalkiten, Natriumphosphat, Kaliumphosphat, Pyridin, Triethylamin und Diisopropylamin.

**5.** Verfahren gemäß einem der vorhergehenden Ansprüche, **dadurch gekennzeichnet, dass** der Umesterungskatalysator ausgewählt wird aus Kaliumcarbonat, Kaliumhydroxid und Lithiumhydroxid.

**6.** Verfahren gemäß einem der vorhergehenden Ansprüche, **dadurch gekennzeichnet, dass** das im Schritt (a) hergestellte Anfangsreaktionsgemisch von 0,1 bis 10 molare Äquivalente, vorzugsweise von 1 bis 5 molare Äquivalente, insbesondere von 1 bis 3 molare Äquivalente des Umesterungskatalysators, bezogen auf die Menge an Dianhydrohexitol, enthält.

**7.** Verfahren gemäß einem der vorhergehenden Ansprüche, **dadurch gekennzeichnet, dass** das Verfahren im Übrigen die Verfolgung der Reaktionsumsetzung umfasst.

**8.** Verfahren gemäß einem der vorhergehenden Ansprüche, **dadurch gekennzeichnet, dass** das Erhitzen im Schritt (b) fortgeführt wird bis zu einem Reaktionsumsetzungsgrad von wenigstens 95%, vorzugsweise wenigstens 98%, idealerweise gleich 100%, wobei der Reaktionsumsetzungsgrad gemäß der folgenden Formel definiert ist:

$$\text{Umsetzungsgrad (in \%)} = \frac{(Ni - Nt)}{Ni} x100$$

worin

*Nt* = Anzahl von Hydroxylfunktionalitäten des im Reaktionsmilieu verbleibenden Dianhydrohexitols zur Zeit t und
*Ni* = Anfangsanzahl von Hydroxylfunktionalitäten des Dianhydrohexitols.

**9.** Verfahren gemäß einem der vorhergehenden Ansprüche, **dadurch gekennzeichnet, dass** das Verfahren im Übrigen wenigstens einen Schritt der Abtrennung des erhaltenen Dianhydrohexitoldialkylcarbonats von einem Teil oder von der Gesamtheit der anderen Komponenten des Reaktionsgemisches umfasst.

**10.** Verfahren gemäß einem der vorhergehenden Ansprüche, **dadurch gekennzeichnet, dass** das Dianhydrohexitol Isosorbid ist.

**11.** Verfahren gemäß einem der vorhergehenden Ansprüche, **dadurch gekennzeichnet, dass** das Dialkylcarbonat Diethylcarbonat ist.

**12.** Verfahren gemäß einem der vorhergehenden Ansprüche, **dadurch gekennzeichnet, dass** das Verfahren im Übrigen einen Schritt (c) des Konzentrierens des (der) im Schritt (b) erhaltenen Dianhydrohexitoldialkylcarbonats (Dianhydrohexitoldialkylcarbonate) durch Verdampfung des im Laufe des Schrittes (b) nicht reagierten Dialkylcarbonats umfasst.

13. Verfahren gemäß Anspruch 12, **dadurch gekennzeichnet, dass** das Verfahren im Übrigen einen Schritt (d) der Reinigung des (der) im Schritt (c) konzentrierten Dianhydrohexitoldialkylcarbonats (Dianhydrohexitoldialkylcarbonate) umfasst.

14. Verfahren gemäß Anspruch 13, **dadurch gekennzeichnet, dass** der vom Schritt (d) der Reinigung stammende Rückstand in das Reaktionsgemisch des Schrittes (a) rückgeführt wird.

15. Dianhydrohexitoldi(ethylcarbonat), insbesondere Isosorbiddi(ethylcarbonat).

16. Isoididdi(methylcarbonat).

17. Isomanniddi(methylcarbonat).

18. Verwendung einer Verbindung gemäß einem der Ansprüche 15 bis 17 oder einer Verbindung, die durch ein Verfahren gemäß einem der Ansprüche 1 bis 14 erhältlich ist, als Monomer oder Comonomer zur Herstellung von Polycarbonaten, Polycarbamaten oder Polythiocarbonaten, vorzugsweise zur Herstellung von alternierenden Polycarbonaten oder Polycarbamaten.

19. Verwendung einer Verbindung gemäß einem der Ansprüche 15 bis 17 oder einer Verbindung, die durch ein Verfahren gemäß einem der Ansprüche 1 bis 14 erhältlich ist, als Mittel zur Kettenverlängerung, Mittel zur Verbrückung, Mittel zur Vernetzung von Polymeren, die Hydroxyl-, Amino-, Thiol-, Ester- oder Oxazolin-Funktionalitäten aufweisen.

**Claims**

1. A process for the preparation of a dianhydrohexitol di(alkyl carbonate) of formula (I)

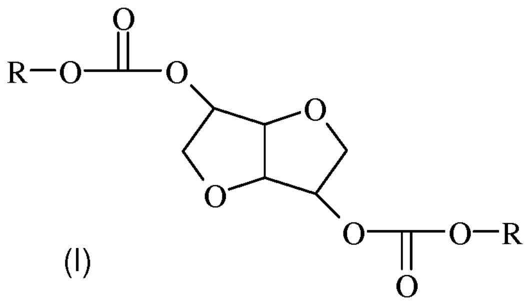

(I)

where each R independently represents a linear or branched alkyl group, preferably a $C_{1-6}$ alkyl group, in particular a methyl or ethyl group,
said process comprising, in sequence, the following steps:

(a) preparation of a starting reaction mixture comprising

- at least one dianhydrohexitol,
- at least 2 molar equivalents, with respect to the amount of dianhydrohexitol present, of at least one dialkyl carbonate of formula R-O-C(=O)-O-R where R has the meaning indicated above, and
- a transesterification catalyst,

(b) heating the reaction mixture up to a temperature of greater than or equal to the boiling point of the alcohol R-OH formed by the transesterification reaction or greater than or equal to the boiling point of the azeotropic mixture which the alcohol R-OH obtained forms with another of the components present in the reaction mixture and at most equal to the boiling point of the reaction mixture, in a reactor equipped with a rectification column comprising a number of theoretical distillation plates sufficient to separate, from the reaction mixture, the alcohol obtained or the azeotrope which it forms with another of the components present in the reaction mixture.

2. The process as claimed in claim 1, wherein the starting reaction mixture comprises from 2.1 to 100 molar equivalents, preferably from 5 to 60 molar equivalents and in particular from 10 to 40 molar equivalents of dialkyl carbonate, with

respect to the amount of dianhydrohexitol initially present in the reaction medium.

3. The process as claimed in claim 1 or 2, wherein the transesterification catalyst is chosen from:

- acid catalysts,
- alkali metal and alkaline earth metal carbonates and hydrogenocarbonates,
- alkali metal and alkaline earth metal hydroxides,
- alkali metal and alkaline earth metal phosphates, hydrogenophosphates and dihydrogenophosphates,
- ammonium salts chosen from ammonium carbonates, hydrogenocarbonates, hydroxides, phosphates, hydrogenophosphates and dihydrogenophosphates, and
- amines.

4. The process as claimed in any one of the preceding claims, wherein the transesterification catalyst is chosen from sulfuric acid, para-toluenesulfonic acid, phosphoric acid, potassium carbonate, sodium carbonate, barium carbonate, cesium carbonate, potassium hydrogenocarbonate, sodium hydrogenocarbonate, lithium hydroxide, potassium hydroxide, sodium hydroxide, calcium hydroxide, hydrotalcites, sodium phosphate, potassium phosphate, pyridine, triethylamine and diisopropylamine.

5. The process as claimed in any one of the preceding claims, wherein the transesterification catalyst is chosen from potassium carbonate, potassium hydroxide and lithium hydroxide.

6. The process as claimed in any one of the preceding claims, wherein the starting reaction mixture prepared in step (a) comprises from 0.1 to 10 molar equivalents, preferably from 1 to 5 molar equivalents and in particular from 1 to 3 molar equivalents of transesterification catalyst, with respect to the amount of dianhydrohexitol.

7. The process as claimed in any one of the preceding claims, wherein the process additionally comprises the monitoring of the progression of the reaction.

8. The process as claimed in any one of the preceding claims, wherein the heating in step (b) is continued up to a degree of progression of the reaction at least equal to 95%, preferably at least equal to 98% and ideally equal to 100%, the degree of progression of the reaction being defined by the following formula:

$$\text{Degree of progression (in \%)} = \frac{(Ni - Nt)}{Ni} x100$$

where

*Nt* = number of dianhydrohexitol hydroxyl functional groups remaining in the reaction medium at time t, and
*Ni* = initial number of dianhydrohexitol hydroxyl functional groups.

9. The process as claimed in any one of the preceding claims, wherein the process additionally comprises at least one step of separation of the dianhydrohexitol di(alkyl carbonate) obtained from a portion or all of the other components of the reaction mixture.

10. The process as claimed in any one of the preceding claims, wherein the dianhydrohexitol is isosorbide.

11. The process as claimed in any one of the preceding claims, wherein the dialkyl carbonate is diethyl carbonate.

12. The process as claimed in any one of the preceding claims, wherein it additionally comprises a step (c) of concentration of the dianhydrohexitol di(alkyl carbonate)(s) obtained in step (b) by evaporation of the dialkyl carbonate which has not reacted during said step (b).

13. The process as claimed in claim 12, wherein it additionally comprises a step (d) of purification of the dianhydrohexitol di(alkyl carbonate)(s) concentrated in step (c).

14. The process as claimed in claim 13, wherein the residue resulting from the purification step (d) is recycled to the reaction mixture of step (a).

**15.** A dianhydrohexitol di(ethyl carbonate), in particular isosorbide di(ethyl carbonate).

**16.** Isoidide di(methyl carbonate).

**17.** Isomannide di(methyl carbonate).

**18.** The use of one of the compounds as claimed in any one of claims 15 to 17 or of a compound capable of being obtained by a process as claimed in any one of claims 1 to 14 as monomer or comonomer in the preparation of polycarbonates, of polycarbamates or of polythiocarbonates, preferably in the preparation of alternating polycarbonates or polycarbamates.

**19.** The use of one of the compounds as claimed in any one of claims 15 to 17 or of a compound capable of being obtained by a process as claimed in any one of claims 1 to 14 as chain-extending agent, bridging agent, grafting agent and crosslinking agent for polymers carrying hydroxyl, amine, thiol, ester or oxazoline functional groups.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 2033981 A
- US 2004241553 A
- JP 6261774 A


### Littérature non-brevet citée dans la description


- **SABER CHATTI.** Cyclic and Noncyclic Polycarbonates of Isosorbide (1,4 :3,6-dianhydro-D-glucitol). *Macromolecules,* 2006, 9061-9070